# EUROPEAN PATENT APPLICATION

(11) **EP 1 589 030 A1**
(43) Date of publication of application: **26.10.2005**
(21) Application number: 04008880.9
(22) Date of filing: 14.04.2004
(51) Int. Cl.: C07K 7/08, C07K 14/47, C12N 5/06, C12N 5/08, C12N 15/11, A61K 38/08, A61K 38/10, A61K 38/17, A61K 45/00, G01N 33/564, G01N 33/574

(54) **Bob-1 specific T cells and methods to use**

(71) Applicant: Friedrich-Alexander-Universität Erlangen-Nürnberg, 91054 Erlangen (DE)
(72) Inventor: Harrer, Ellen G., Dr., 91054 Erlangen (DE); Harrer,Thomas, Prof.Dr., 91054 Erlangen (DE)
(74) Representative: Fleuchaus, Andrea

(57) **Abstract**

The present invention refers to the use of Bob-1 protein, fragments or epitopes thereof and/or related sequences in a method to identify, to induce and/or to isolate Bob-1 specific or Bob-1 cross-reactive T-cells, preferably cytotoxic T-lymphocytes (CTL) and CD4⁺ T-helper cells. The T-cells according to the invention can be used to identify and diagnose various diseases, which are influenced by Bob-1 protein expression. The invention provides further the use of Bob-1 specific and/or Bob-1 cross-reactive T-cells, as well as peptides and/or nucleic acid sequences for inducing Bob-1 specific and/or Bob-1 cross-reactive T-cells, as medicament or vaccine and for the treatment of Bob-1 expressing tumors, lymphomas and autoimmune diseases.

## Description

The present invention refers to the use of Bob-1 protein, fragments or epitopes thereof and/or related sequences in a method to identify, to induce and/or to isolate Bob-1 specific or Bob-1 cross-reactive T-cells, preferably cytotoxic T-lymphocytes (CTL) and CD4⁺ T-helper cells. The T-cells according to the invention can be used to identify and diagnose various diseases, which are influenced by Bob-1 protein expression. The invention provides further the use of Bob-1 specific and/or Bob-1 cross-reactive T-cells, as well as peptides and/or nucleic acid sequences for inducing Bob-1 specific and/or Bob-1 cross-reactive T-cells, as medicament or vaccine and for the treatment of Bob-1 expressing tumors, lymphomas and autoimmune diseases.

### Background of the Invention

Bob-1 protein, also known as OBF-1 or OCA-B, was initially identified as a B-cell restricted coactivator (Swissprot, Accession No. Q16633), which interacts with the octamer binding transcription factors, Oct1 and Oct2, increasing the transcriptional activity at the octamer motif-containing promoters. Such octamer motif-containing promoters are primarily found in B-cell-specific immunoglobulin (Ig) gene promoters.

It was described that Bob-1 itself does not bind directly to DNA, but that transcriptional activity at octamer motif-containing promoters requires a functional interaction of the Oct1 or Oct2 transcription factor with at least one specific coactivator, e.g. Bob-1. Since octamer motives are conserved in virtually all immunoglobulin (Ig) gene promoters it is believed that Bob-1 is essential for Ig gene expression.

Furthermore, recent studies revealed that Bob-1 is also involved in all stages of B-cell development and particularly in the formation of germinal centers.

It has been shown that octamer-dependent transcription in pre-B-cell lines derived from Bob-1-deficient mice demonstrates that Bob-1 is an essential and non-redundant component of transcription at octamer-motive containing promoters (Laumen, H., P. J. Nielsen, and T. Wirth. 2000. Eur. J. Immunol. 30: 458-469).

Given the fact that Bob-1 deficient mice have serious defects in antigen-dependent B-cell differentiation, the normal expression of Bob-1 in early B-cell populations and germinal centers, the preponderance of octamer motifs in Ig gene promoters, as well as the essential role of Bob-1 in octamer-dependent transcription, Bob-1 could be expected to play an essentially role in all antigen-driven stages of B-cell maturation and activation.

Bob-1 protein, its coding sequence, a vector comprising the coding sequence and a composition comprising the protein are known from WO 95/32284. Bob-1 has been suggested for the production of specific anti-Bob-1-antibodies, which might be useful in the localization of Bob-1 expression in the tissue. WO 95/32284 further mentions in general terms the use of isolated Bob-1 protein as replacement therapy for disorders or diseases where Bob-1 expression is lacking.

While it was expected that a lack of Bob-1 expression can lead to serious effects for the normal B-cell development and activity, including immunological defects and related disease, it was not clear for long time whether also an up regulation of Bob-1 expression would cause any diseases or disorders.

Only recently Greiner et al. (American Journal of Pathology 2000, 156: 501-507) showed that more than 90% of Burkitt's lymphomas, follicular lymphomas or highly malignant lymphomas as well as above 50% of the examined B-cell lymphomas show significant over-expression of the Bob-1 protein.

The term "lymphoma" as used in the context of the present invention comprises all B-cell- or T-cell-derived benign or malignant cancers of the haemopoietic system. Particularly, the term lymphoma summarizes Hodgkin Lymphoma (HL) and Non-Hodgkin Lymphomas (NHL), such as Burkitt, follicular or diffuse lymphoma. In the context of the present invention the term lymphoma further refers particularly to such variants of lymphomas, which express or over-express Bob-1 protein.

The term "over-expression" as used herein describes any non-physiological value in the Bob-1 protein expression, either in the amount of Bob-1 protein expression or in the time schema of Bob-1 protein expression.

So far there is no adequate treatment available for the majority of Non-Hodgkin Lymphomas (NHL), which occur with an increasing incidence of 8-18/100.000 people per year, and a subset of Hodgkin Lymphoma. Hodgkins and Non-Hodgkins lymphomas are still treated with combination therapies comprising chemotherapy, radiotherapy, bone marrow transplantation and treatment with specific antibodies.

Up to 90 % of patients with limited Hodgkin Lymphoma, but only approximately 50% of patients with disseminated Hodgkin Lymphoma and, depending on subtypes and risk factors, only up to 50% of high-grade lymphoma can be cured by an aggressive chemotherapy. Chemotherapy causes substantial side effects and also long-term toxicity such as infertility in surviving patients. Treatment options are limited especially in elderly people and patients with concomitant diseases. In contrast, except for rare subtypes such as hairy cell leukemia low grade Non-Hodgkin Lymphoma cannot be cured by chemotherapy. Some subtypes of Non-Hodgkin Lymphoma, such as Grade I and II follicular lymphoma can be cured in early stages (stages I to II of the Ann-Arbor Classification) by radiation therapy, but there is no curative therapy in advanced stages of diseases. Unfortunately, the majority of the low-grade lymphomas is diagnosed in a stage when a curative therapy is not available any more. Thus, current treatment modalities such as chemotherapy or immunotherapy can achieve only transient remissions or palliation. This is the reason that chemotherapy in many subsets of low grade Non-Hodgkin Lymphomas such as Grade I and II follicular lymphomas or chronic lymphocytic leukemia is deferred until clinical symptoms emerge.

A therapeutic strategy for the treatment of HD and NHD lymphoma has been recently offered by the use of Rituxan® from Genentech, which is a specific B-cell destroying antibody, and the medicament Zevalin® from IDEC, which is a radioactively labeled monoclonal antibody, which also depletes B-cells. The main disadvantage of these antibody treatments is that the antibodies destroy all and every B-cell in a patient, in the germinal centers as well as in the periphery. Accordingly, this therapy can induce a decrease in immunoglobulin, which then must be treated by infusions with immunoglobulin to avoid immunodeficiency.

### Object of the Invention

It is therefore an object of the present invention to treat lymphomas, such as HL and NHL, and further tumors or lymphomas, which are characterized by an expression or over-expression of Bob-1 protein.

It is particularly an object of the present invention to improve the efficacy of treatment of malignant lymphomas and at the same time reduce the side effects, which are one major burden of conventional therapies.

It is furthermore an object of the present invention to provide means and methods to treat autoimmune diseases and disorders, which are characterized by the production of auto-antibodies correlated with an expression or over-expression of Bob-1 protein.

Additionally, it is an object of the present invention to provide means and methods to diagnose or also newly identify disorders or diseases, which are dependent on or influenced by recognition of physiological or non-physiological levels of Bob-1 protein expression.

### Detailed description of the Invention

In this context it can be regarded as outstanding success of the inventors, that they were able to identify T-cells (TC), particularly cytotoxic T-lymphocytes (CTLs) and CD4⁺ T-helper cells, which are specifically directed to or had a highly specific cross-reactivity with Bob-derived peptides presented by HLA molecules on the cell surface of Bob-1 expressing cells.

The inventors, however, not only identified T-cells (TC), particularly cytotoxic T-lymphocytes (CTLs) and CD4⁺ T-helper cells, which are both a subfraction of the overall T-cell population and which are according to the invention specifically directed to or had a highly specific cross-reactivity with Bob-derived peptides, but the inventors were furthermore able to induce, generate and isolate such Bob-1 specific and Bob-1 cross-reactive T-cells.

The term "T-cells" or "T-lymphocytes" is understood herein to describe cells deriving from the haemopoietic system; mainly small, agranulocytic leukocytes that normally make up a quarter of the white blood cell count, that develop in the thymus and that may increase in the presence of infection or malignancy. The principal functions of T-cells are to regulate all immune responses to protein antigens and to serve as effector cells for the elimination of intracellular microbes. There are various subspecies of T-cells, like CD4⁺ T-helper cells (CD4⁺ cells), cytotoxic T-cells (CTLs) or gamma-delta T-cells, which are normally characterized by defined surface structures, like e.g. CD4 antigen for helper T-cells or CD8 antigen for cytotoxic T-cells.

The term "cytotoxic" and alternatively "cytolytic" is understood in the context of the application as CD8 positive T-cells, which recognize peptide antigens bound to HLA class I molecules on the surface of a cell. Such peptide antigens presented on the surface of the cell are derived from processed proteins synthesized within the cytoplasm of the cell. After specific interaction of the processed peptide antigens with their T-cell receptors, they will be presented bound to HLA class I molecules on these cells. The cytotoxic T-cells are able to kill such peptide antigen presenting cells.

CD4⁺ T-helper cells specifically recognize peptide antigens bound to HLA class II molecules on the surface of antigen-presenting cells. These peptides usually are derived from exogenous antigens taken up and processed by professional antigen presenting cells such as dendritic cells (DC), macrophages and B-cells. After recognition of the antigen CD4⁺-T-helper cells become activated and provide important help to CTL and other immune effector cells such as B-cells, macrophages and NK-cells by secreting a variety of cytokines and chemokines. It has been shown in animal models, both for malignant and for infectious diseases, that an optimal immune response is generated by stimulating both antigen-specific CD8⁺ CTL and CD4⁺ T-helper cells.

With these highly specific TC, both CTLs and CD4⁺ T-helper cells, the inventors had for the first time a tool in their hands to diagnose diseases characterized by an immune reaction against Bob-1 expressing cells.

Additionally, with the knowledge of the inventors it became possible for the first time to generate *in vitro* but also to induce *in vivo* TC, both CTLs and CD4⁺ T-helper cells, specific for Bob-1 protein.

The artificially generated or induced T-cells according to the invention provide highly specific means to treat disorders or diseases correlated with expression or over-expression of Bob-1 protein.

For example such Bob-1 specific CTL specifically recognize cells, which present Bob-1 fragments or epitopes bound to receptors on the surface. They further specifically bind to such Bob-1 expressing cells, and have than the capacity to destroy or kill such Bob-1 expressing cells. A typical, but non limitating example of such Bob-1 expressing cells are B-cells deriving from a malignant lymphoma or B-cells producing auto-antibodies.

As a further example, Bob-1 specific CD4⁺ T-helper cells (alternative nomenclature is CD4⁺-cells or CD4⁺) are getting activated after recognition of Bob-1 derived peptides presented by antigen presenting cells. Activated Bob-1 specific CD4⁺-cells can provide important help to boost the activity of CTL and other immune effector cells such as NK-cells or macrophages to kill Bob-1 expressing cells.

It was, therefore, the extraordinary achievement of the inventors to provide means and methods, which are capable to activate or induce T-cells with a high specificity or cross-reactivity to Bob-1 protein.

For this the present invention provides peptides, which derive from or are related to epitopes of the Bob-1 protein or a modified Bob-1 protein.

Several peptides preferably stimulating Bob-1 specific T-cells, preferentially CTL and/or CD4⁺ T-helper cells are provided.

According to one embodiment the group of peptides able to induce Bob-1 specific CTL can be summarized by the general sequence

X₁X₂KX₃PX₄X₅X₆X₇,

whereby
X₁ is selected from the group comprising any naturally occurring amino acid, particularly Arginine (R), Isoleucine (I), Phenylalanine (F), Tyrosine (Y) or Serine (S) is,
X₂ is Methionine (M), Valine (V), Leucine (L) or Isoleucine (I),
X₃ is Glutamic acid (E) or Aspartic acid (D),
X₄ is selected from the group comprising any naturally occurring amino acid particularly Valine (V), Leucine (L), Isoleucine (I), or Alanine (A) is,
X₅ is selected from the group comprising any naturally occurring amino acid particularly Histidine (H) or Leucine (L),
X₆ is selected from the group comprising any naturally occurring amino acid particularly Glutamic acid (E) or Glycine (G), and
X₇ is Methionine (M), Valine (V), Leucine (L), Isoleucine (I) or Alanine (A).

According to a further embodiment peptides of this group are HLA A2 binder and therefore have preferably the amino acids M, L, V or I at the position X₂ and the amino acids V, L or M at position X₇.

According to a still further embodiment of the present invention the peptide for the induction of Bob-1 specific or Bob-1 cross-reactive TC, CTLs or CD4⁺ is selected from the group comprising the peptides FLKEPVKEV, SLKEPVKEL, YLKEPVKEL, FLKEPVKEL, ILKEPVHEV, ILKEPVHGV, RVKEPVKEL, DSDAYALNHTLSVEGF, AYALNHTLSVEGF, YALNHTLSVEGF, YALNHTLSVEG, ALNHTLSVEGF, TYASPPLITNVTTRSSATPA or AALCAGWLSQPTPATLQPLA.

It is particularly interesting that all these sequences have as a common characteristic that they induce T-cells, which are either specific to Bob-1 protein or are highly cross-reactive to Bob-1 protein.

In the context of this invention a T-cell, which is specific, binds the particular peptide in its binding groove. The binding groove is formed in a way to bind only a particular peptide having the amino acid sequence, which is particularly recognized by the specific T-cell. The binding is considered specific, if due to the binding the T-cell is activated. Activation normally leads to amplification of the specific binding T-cells.

Surprisingly, not only peptides derived from Bob-1 protein, are capable of inducing Bob-1 specific T-cells. Interestingly, the inventors were able to identify peptides from unrelated proteins, which however are able to induce T-cells, which interact specifically with Bob-1 protein. For such T-cells, which are induced by a peptide, which is not derived from Bob-1 protein, but from either a modified Bob-1 protein or any unrelated protein, the term "cross-reactive" to Bob-1 protein is used in the context of the present invention.

Particularly useful is the peptide FLKEPVKEV, which is derived from a modified Bob-1 protein and which surprisingly showed significantly better T-cell-inducing capacity than a Bob-1 derived peptide, e.g. RVKEPVKEL.

Regarding the peptides it was further highly surprising, that also peptides, which derive from completely unrelated sources such as from HIV, namely ILKEPVHEV, ILKEPVHGV or ILKDPVHGV, can be used to induce Bob-1 specific or cross-reactive T-cells, preferable CTLs.

Another example for Bob-derived T-cell inducing peptides is the peptide ALNHTLSVEGF, which is able to stimulate Bob-1 specific and/or cross-reactive T-cells, preferable CD4⁺-cells. As further examples also slightly modified versions of the ALNHTLSVEGF peptide, namely the peptides DSDAYALNHTLSVEGF, AYALNHTLSVEGF, YALNHTLSVEGF, YALNHTLSVEG, are highly useful to stimulate and to generate Bob-1 specific and/or cross-reactive T-cells, preferable CD4⁺-cells. These peptides are HLA class II restricted and are presented preferably on HLA DQ molecules, more preferably on HLA DQ0604 molecules. Additionally, these peptides can also be presented on HLA DP, preferable HLA DP04 molecules.

It was shown that out of the peptides derived from the peptide DSDAYALNHTLSVEGF, the peptide ALNHTLSVEGF has the highest capacity to induce Bob-1 specific or cross-reactive T-cells. The peptides YALNHTLSVEGF and YALNHTLSVEG are still recognized, but the induction of T-cells is less effective than an induction with ALNHTLSVEGF.

According to still a further example also the peptides TYASPPLITNVTTRSSATPA, AALCAGWLSQPTPATLQPLA and fragments with nine or more amino acids of these peptides are highly useful to stimulate and to generate Bob-1 specific and/or cross-reactive T-cells. These peptides are particularly useful to induce CD4⁺, but also CD8⁺ cells in patients,

All the above-mentioned peptides are particularly useful for the identification, but also the induction and isolation of T-cells, which are specific for or cross-reactive to epitopes or fragments of the Bob-1 protein.

For this, particularly for an induction of T-cells, which are specific for or cross-reactive to epitopes or fragments of the Bob-1 protein, the peptides according the invention will be used to stimulate a population of mixed lymphocytes, preferably peripheral blood mononuclear cells (PBMC). Such mixed lymphocytes or PBMCs can be isolated from the blood of a patient according to well-known standard technologies, e.g. Ficoll-Hypaque density gradient centrifugation (Pharmacia, Uppsala, Sweden). The isolated mixed lymphocytes or PBMCs are then incubated for stimulation with peptides according to the invention and, optionally, also with immune hormones, like e.g. interleukin-2. After two weeks, stimulated T-cells outgrow the culture of mixed lymphocytes. Outgrowing antigen-specific cells can be identified by analysis of antigen-induced cytokine secretion on a single cell level using a standard ELISPOT assay. By this ELISPOT assay the number of antigen-specific T-cells can be quantified by video-based analysis of spot forming units within cell cultures.

The term "spot" or "spot forming unit" in the context of this invention refers to T-cells which secrete gamma-IFN after stimulation with antigens such as synthetic peptides, proteins or cells. The accumulation of cells in the context of T-cell induction is due to a specific outgrow of antigen-stimulated cells and thus an increased number of cells. Such accumulation can be identified under the microscope and can be stained with well-known technologies. Each spot corresponds to an individual T-cell clone, which has been stimulated and induced according to the invention and is thus Bob-1 specific or cross-reactive.

The outgrowing cells were tested with standard assays, like chromium-release assay or ELISPOT assay, for specific recognition of or cross-reactivity to Bob-1 epitopes. For example, in the ELISPOT assay the readout for specific recognition or cross-reactivity to Bob-1 is correlated with the amount of released interferon-gamma, which is detected with an interferon-gamma specific antibody (e.g. 1-D1K: Mabtech, Stockholm, Sweden). This method allows the sensitive detection of antigen-specific T-cells on a single cell level.

It could be shown by the inventors that by stimulation according to the invention it is possible and surprisingly efficient to outgrow T-cells, which are specific for or cross-reactive to epitopes or fragments of the Bob-1 protein.

If T-cells, which are specific for or cross-reactive to epitopes or fragments of the Bob-1 protein are needed for further experiments or any therapeutic strategy, they can be isolated e.g. by FACS sorting using soluble HLA-peptide complexes such as HLA-tetramers (Proimmune, Oxford, UK) or HLA-streptamers (IBA GmbH, Göttingen, Germany) carrying the specific peptides or by the gamma-IFN-secretion assay (Miltenyi, Bergisch-Gladbach, Germany). Accordingly, the invention also provides isolated TC, both CTLs and CD4⁺ T-helper cells, which are specific to or cross-reactive with Bob-1 protein, fragments or epitopes thereof or at least one of the peptides according to the invention.

The Bob-1 specific or cross-reactive TCs, according to the invention are particularly useful to treat patients with tumors or lymphomas, which are characterized by Bob-1 expression or Bob-1 over-expression.

The general principle behind such treatment refers back to specificity or cross-reactivity of the TCs, CD4+ or CTLs, to Bob-1 epitopes or fragments, which are presented on cells and which will be found and eventually eliminated by the Tcs of the invention. Particularly, in patients suffering from an uncontrolled growth of tumor cells or lymphocytes, accordingly patients having a lymphoma, it is highly desirable to destroy and eliminate such uncontrolled growing tumor cells or lymphocytes.

Since it was shown that many tumors and most lymphomas show an increased Bob-1 expression, the TCs according to the invention are highly useful to eliminate such Bob-1 expressing cells.

The TCs according to the invention have one additional advantage compared with other therapeutic strategies destroying lymphoma cells. While the known antibodies from Genentech (Rituxan) or IDEC (Zevalin) do eliminate, due to a general specificity for B-cells, all B-cells in a patient, in the periphery as well as in the germinal centers of the lymph nodes. In contrast to this the TCs, CD4⁺ or CTLs, according to the invention only eliminate cells with an increased Bob-1 expression. Since it is known that primarily the B-cells in the germinal centers express Bob-1, but not the B-cells in the periphery, and since it is further known that many malignant lymphomas over-express Bob-1 the TCs of the invention do not deplete every and any B-cell in a patient, but preferably eliminate cells expressing or over-expressing Bob-1 protein.

Consequently, the side effects of the method of treatment according to the invention are clearly reduced compared to any so far known therapeutic strategies.

Even in cases where beside lymphoma cells also some normal B-cells might express Bob-1 a treatment with the TCs according to the invention is highly advantageous, because the ratio of the eradication of malignant tumor or lymphoma cells compared to normal B-cells will be very favorable for the patient. Additionally, in cases where depletion of B-cells could induce a significant decline of antibody levels in the serum, it is easy and generally accepted to additionally treat such patients with commercially available immunoglobulin preparations to correct this humoral immune defect.

The invention further provides isolated TCs, both CD4⁺ T-helper cells and CTLs, useful in a method to treat tumors or lymphomas, particularly lymphomas, which are characterized by an increased expression of Bob-1 protein.

For this, firstly, PBMC are isolated from a patient, these PBMC will be induced with at least one of the peptides according to the invention, with Bob-1 protein, fragments or epitopes thereof or with modified Bob-1 protein fragments, or epitopes thereof to outgrow Bob-1 specific or Bob-1 cross-reactive TCs, both CTLs and CD4⁺ T-helper cells.

Optionally, immune hormones are added to the induction reaction. Typically, interleukin-2, IL-7, gm-CSF, IL-12, IL-18 and alpha-IFN are used as immune hormones.

The outgrowing T-cells are then isolated or at least enriched by known standard technologies. For the method to treat such isolated or enriched cells are then re-administered to the patient. Typical ways of administering are intradermally, subcutaneously, intramuscularly, intravenously or as intralymphatic injection.

The re-administered enriched TCs supported by the activity of specific Bob-1 specific T-helper cells, will efficiently eliminate Bob-1 expressing tumor and/or lymphoma cells.

Additional immune therapies or immune replacement therapies may be combined with the above-described method of treatment.

While it is preferred to use PBMC of the individual patient for the treatment as described above, it is also possible to use allogeneic PBMC, accordingly PBMC of a different patient, to outgrow Bob-1 specific TC, CD4⁺ cells or CTLs, which than can be relocated to any patient. In this case it is advisable to control any possible immune rejection of the relocated TCs according to standard practice in transplantation medicine.

Alternatively, in another embodiment of the invention patients with tumors or lymphomas, which are characterized by increased Bob-1 expression, are treated by a method for *in vivo* induction of Bob-1 specific or cross-reactive TCs, CTLs or CD4⁺-cells.

For this the patient is inoculated with at least one of the peptides of the invention, a Bob-1 protein, a fragment or epitope thereof or a modified Bob-1 protein, a fragment or epitope thereof to induce *in vivo* T-cells, which are specific for or cross-reactive to epitopes or fragments of the Bob-1 protein or the modified Bob-1 protein.

In this method of treatment the peptides according the invention will be directly administered to a living animal or human. The peptides are preferably, but not limitatingly, administered intracutaneously, subcutaneously, intramuscularly or intranasally.

Typical dosages for immunization may range from about 0.001 to about 1 protein or peptide per kg body weight (mg/kg), or more particularly from about 0.01 to about 0.1 mg/kg. Preferably doses are within a range of 50 µg to 1 mg of the protein or peptide per application.

Optionally, immune hormones, adjuvants or factors that block an immune suppression are additionally administered to provoke an increased reaction to the peptides, or protein fragments. For enhanced efficacy of the immunization, it is useful to repeat the application of the peptides 2 to 3 times at 2 to 8 week time intervals.

Furthermore, and according to well-known experience in vaccine development the induction of Bob-1 specific CTL will be enhanced by parallel induction of Bob-1 specific CD4⁺-cells. Accordingly, it is highly interesting for the method of treatment to additionally or alternatively induce Bob-1-specific CD4⁺-cells. This can be achieved by administering to a patient the whole Bob-1 protein or shorter amino acids and peptides thereof with a length of at least 10 amino acids. The proteins or peptides can be administered intracutaneously, subcutaneously, intramuscularly or intranasally. The proteins or peptides will be taken up by antigen presenting cells. These cells will present the proteins or peptides on their receptors to stimulate immune cells, particularly CD4⁺-cells. Stimulation of immune cells leads to an outgrow of the particularly stimulated cell type.

Typical dosages for immunization may range from about 0.001 to about 1 mg protein or peptide per kg body weight (mg/kg), or more particularly from about 0.01 to about 0.1 mg/kg. Preferably doses are within a range of 50 µg to 1 mg of the protein or peptide per application.

Optionally, immune hormones, adjuvants or factors that block an immune suppression are additionally administered to provoke an increased reaction to the peptides, or protein fragments. For enhanced efficacy of the immunization, it is useful to repeat the application of the peptides 2 to 3 times at 2 to 8 week time intervals.

Bob-1 specific CD4⁺ T-helper cells can provide help not only to Bob-1 specific CTL, but also to other CTL with specificity to other tumor antigens derived from the same tumor cell.

In accordance with the above-described methods, the invention provides further a composition, alternatively a pharmaceutical composition, comprising one or more of the peptides of the invention, a Bob-1 protein, a fragment or epitope thereof or a modified Bob-1 protein, a fragment or epitope thereof and, optionally, at least one pharmaceutical acceptable carrier or diluent.

Typically, such "pharmaceutically acceptable carrier" are carriers and diluents selected so as not to significantly impair biological activity of the agent (e.g. binding specificity, affinity or stability), such as water, saline, Ringer's solution, dextrose solution, 5% human serum albumin, fixed oils, ethyloleate, or liposomes. Acceptable carriers may include biocompatible, inert or bio-absorbable salts, buffering agents, oligo- or polysaccharides, polymers, viscoelastic compound such as hyaluronic acid, viscosity-improving agents, preservatives, and the like. In addition, the pharmaceutical composition or formulation may also include other carriers, adjuvants, or nontoxic, non-therapeutic, non-immunogenic stabilizers and the like.

Furthermore, the invention provides a vaccine comprising one or more of the peptides of the invention, a Bob-1 protein, a fragment or epitope thereof or a modified Bob-1 protein, a fragment or epitope thereof and, optionally, one or more immune hormones, adjuvants or factors blocking an immune suppression.

The vaccine can be administered intradermally, subcutaneously, intramuscularly, intravenously, intranasally or as intralymphatic injection.

Typical dosages for vaccination ranges from about 0.001 to about 1 mg protein or peptide per kg body weight (mg/kg), or more particularly from about 0.01 to about 0.1 mg/kg.

Typically, the optionally administered immune hormones, adjuvants or factors blocking an immune suppression are cytokines, like interleukin-2, interleukin-18, interferon-gamma, interferon-alpha or gm-CSF, or immunostimulatory molecules such as RNA-molecules (for examples Poly-I-Poly-C (Ampligen) or capped and polyadenylated messenger RNA such as RNActive™ from CureVac GmbH, Tübingen, Germany) or short DNA-molecules such as CPG-oligonucleotides (for example CPG 7909, currently used in clinical vaccine trials by Coley Pharmaceutical Group, Wellesley, MA, USA).

According to a further embodiment of the invention, patients having a tumor or lymphoma, which shows an increased expression of Bob-1 protein, can be treated by directly administering to the patient the nucleic acid sequence, which encodes alone or in combination one or more of the peptides of the invention, the Bob-1 protein, fragments or epitopes thereof or a modified Bob-1 protein, fragments or epitopes thereof. According to this method such sequences will be taken up by dendritic cells and monocytes. Subsequently, the sequences will be translated into peptides or proteins, which then will be presented via MHC I and MHC II complexes to the immune system and particularly to TCs. The presentation of these specific peptides or protein fragments will induce TC stimulation and, thus, an outgrowth of the TCs, namely CD4⁺ or CTLs, which are specific for or cross-reactive with Bob-1 protein. These specific TCs will subsequently destroy tumor or lymphoma cells, which express Bob-1 protein.

As also mentioned above, an additional immunoglobulin replacement therapy to compensate severe immune defects is optionally applicable.

The nucleic acid sequence according to the invention is either a DNA or RNA molecule, which encodes alone, in combination one or more of the peptides of the invention, the Bob-1 protein, fragments or epitopes thereof or a modified Bob-1 protein, fragments or epitopes thereof.

In another example the DNA or RNA molecule encodes connected with and/or without linkers between the single nucleic acid molecules one or more of the peptides of the invention, the Bob-1 protein, fragments or epitopes thereof or a modified Bob-1 protein, fragments or epitopes thereof.

The nucleic acid sequences can be administered intradermally, subcutaneously, intramuscularly or intranasally, as naked DNA, as DNA contained in particles such as liposomes, as DNA contained in plasmids or as DNA contained in vectors such as recombinant vaccinia viruses or recombinant MVA-vectors (Modified Vaccinia Virus Ankara). In addition, it is possible to apply the DNA in suitable carriers directly on the skin. The nucleic acid sequences also can be applied in form of RNA molecules, which have been chemically modified to avoid degradation by Rnases (for example capped and polyadenylated messenger RNA such as RNActive™ from CureVac GmbH, Tubingen, Germany).

According to a further embodiment a patient in need thereof can be vaccinated by ex-vivo generated dendritic cells. Dendritic cells can be generated by incubation of monocytes or hematopoetic stem cells with cocktails of cytokines including gm-CSF, IL4, TNF-alpha, prostaglandin E2 and IL-6. Maturating dendritic cells can be loaded with peptides, proteins, nucleic acids such as DNA or RNA-molecules, according to the invention, and after maturation they can be administered to patients. It is possible both to use autologous and also allogeneic HLA-matched dendritic cells.

In accordance with the above, the invention therefore provides a vaccine comprising one or more of the peptides of the invention, the Bob-1 protein, fragments or epitopes thereof or a modified Bob-1 protein, fragments or epitopes thereof, one or more nucleic acid sequence of the invention and, optionally, a pharmaceutical acceptable carrier, diluent, one or more immune hormones, one or more adjuvants or one or more factors blocking an immune suppression.

For the preparation of a vaccine the peptides, proteins or nucleic acid sequences according to the invention are converted into a physiologically acceptable form.

Therefore, typically, about 1000 µg of the peptides, proteins or nucleic acid sequences are freeze-dried in 1 ml of phosphate-buffered saline (PBS), optionally, in the presence of 2% peptone and 1% human albumin in an ampoule, preferably a glass ampoule. The lyophilisate can contain extenders (such as mannitol, dextran, sugar, glycine, lactose or polyvinylpyrrolidone) or other aids (such as antioxidants, stabilizers, etc.) suitable for parenteral administration. The glass ampoule is then sealed and can be stored, preferably at temperatures below -20°C., for several months. For vaccination or therapy the lyophilisate can be dissolved in 0.1 to 1 ml of an aqueous solution, preferably physiological saline, and administered either parenterally, for example by intramuscular inoculation or locally. Vaccines or therapeutics according to the invention are preferably injected intramuscularly or subcutaneously, but dependent on the carrier vaccines can be applied also by other routes such as epidermally, intradermally or intranasally in the case of DNA - or RNA vaccines, intradermally in the case of dendritic cells or vectors such as MVA, by the oral or nasal route in the case of recombinant vectors or subcutaneously or intravenuously in the case of dendritic cells.

The mode of administration, the dose and the number of administrations can be optimized by those skilled in the art in a known manner. It is expedient where appropriate to administer the vaccine several times over a lengthy period in order to obtain appropriate immune responses against the agents according to the invention.

According to still a further embodiment the invention provides methods to diagnose diseases or disorders correlated with a Bob-1 expression. For this, Bob-1-specific T-cells will be detected within PBMC or within suspension of lymph nodes by several methods. According to one examplatory method, short peptides corresponding to optimal T-cell epitopes such as the peptide RVKEPVKEL can be used for the generation of soluble HLA-complexes, which are able to bind specifically to Bob-1-specific TCR and, thus, allow direct and specific staining of Bob-1-specific T-cells. Examples for commercially available HLA-complexes are HLA-tetramers and HLA-pentamers (Proimmune, Oxford, UK), HLA-streptamers (Iba GmbH, Göttingen), HLA-Dimers (Beckton Dickinson, Franklin Lakes, NJ, USA) and soluble HLA-molecules assembled with peptides and a β-2-microglobulin molecule, which is linked to stain molecules, such as phycoerythrin.

Another diagnostic method is the incubation of lymphocytes such as PBMC isolated from a patient with one or more of the peptides according to the invention or a Bob-1 protein, fragment or epitope thereof. After an incubation time that allows the activation and/or outgrowing of specific T-cells, the activation or outgrow of Bob-1-specific T-cells is analyzed in appropriate assays, preferably an ELISPOT assay, but other assays such as intracellular cytokine staining assays (such as the assay distributed by Beckton Dickinson, Franklin Lakes, NJ, USA) or cytokine secretion assays (such as the assay distributed by Miltenyi Biotec GmbH, Bergisch-Gladbach, Germany), chromium release assays, lymphoproliferation assays or direct staining assays using soluble HLA-peptide complexes such as HLA-tetramers, HLA-pentamers, HLA-Dimers or HLA-streptamers can be used, too.

While Bob-1 specific T-cells usually cannot be detected in circulating PBMC in healthy persons, it is clearly possible with the methods of the invention to identify and describe Bob-1-specific TCs in a patient and thus diagnose and describe a correlated Bob-1 dependent disorders or diseases.

The diagnostic methods not only can be used for the diagnosis of Bob-1 associated tumors, lymphomas or autoimmune diseases, but also for the monitoring of Bob-1 specific T-cells after applications of vaccines with the purpose to induce Bob-1 specific T-cells such as in patients in need thereof.

According to a further embodiment the invention provides a kit for diagnostic usage. Such kit comprises one or more of the peptides of the invention, the Bob-1 protein, fragments or epitopes thereof or a modified Bob-1 protein, fragments or epitopes thereof, or one or more nucleic acid sequence of the invention packed in a way suitable for contacting these means in a container with the PMBC isolated from a patient.

According to further embodiment the diagnostic method and kit are also particularly useful for the diagnosis of Bob-1 associated autoimmune disorders or diseases.

Furthermore, according to another embodiment the peptides, proteins, protein fragments, epitopes or sequences according to the invention are also particularly useful for the treatment of autoimmune disorders or diseases in general.

"Auto-immune disorders or diseases" in the context of this application comprise diseases characterized by the production of self-reactive T-cells and/or B-cells recognizing with their T-cell receptor (TCR) or their B-cell receptor, respectively, autologous structures of the body. A variety of autologous tissues or autologous molecules can be targeted by autoreactive T-cells or B-cells such as pancreas in the case of diabetes mellitus, myelin basic protein in the case of multiple sclerosis, DNA in the case of systhemic Lupus erythematodes, proteinase 3 in the case of Morbus Wegener, the TSH - receptor in the case of Morbus Basedow. Autoreactivity by T-cells can lead to destruction of target cells and tissues or to inflammation due to the release of a variety of cytokines by autoreactive T-cells. Autoreactivity by B-cells usually is associated by the production of auto-antibodies binding to autologous molecules thereby inducing damage such as cell death, induction of inflammation, receptor blockade or even unphysiological receptor triggering such as in the case of TSH-receptor binding antibodies in Morbus Basedow.

For the treatment of autoimmune disorders or diseases the peptides, proteins, protein fragments, epitopes or sequences according to the invention are used to induce Bob-1 specific T-cells, which then destroy B-cells producing antibodies or immunoglobulin directed against self-antigens. Alternatively, autologous T-cells could be induced and increased *ex vivo* and subsequently administered to a patient in need thereof. Thus, the antibody production is decreased and symptoms related to auto-antibodies diminish. Side effects of such treatment can be easily compensated with an additional and well-known immunoglobulin replacement therapy.

The feasibility of such an approach to induce an humoral immunodeficiency for the treatment of autoimmune diseases is demonstrated by the inventors in a male patient suffering from a severe MCTD (mixed connective tissue disease) with pulmonary fibrosis, who was cured from this autoimmunity after he developed a common variable immunodeficiency (CVID) associated with a decrease of his serum immunoglobulin levels and a concomitant loss of his auto-antibodies such as antinuclear antibodies or n-RNP-antibodies.

In this patient the inventors could further detect the presence of Bob-1 specific T-cells recognizing both the HLA-class II - restricted peptide DSDAYALNHTLSVEGF and the HLA-class I - restricted peptide RVKEPVKEL. The inhibition of immunoglobulin production by destruction of Bob-1-expressing B-cells by his Bob-1-specific T-cells could suppress the production of autoantibodies and cure his life threatening rheumatic disease. Except for substitution of immunoglobulins he does not need anymore any immunosuppressive therapy for his mixed connective tissue disease. This patient demonstrates that the development of Bob-1-specific T-cells can cure autoimmune diseases caused by the production of auto-antibodies.

In this context it has been furthermore shown by the inventors that in humans an autoimmune reaction against B-cells with specific recognition of Bob-1 by Bob-1-specific T-cells can lead to an autoimmune disease characterized by the induction of an immunodeficiency due to a lack of antibodies caused by the destruction of Bob-1 expressing B-cells.

It is the achievement of the inventors to delineate this pathogenesis of an important subgroup of patients suffering from the Common Variable Immunodeficiency (CVID) with its pathogenic reason of B-cell destruction. This immunodeficiency, which usually emerges in patients in an age of 20 to 40 years, is characterized by a progressive loss of immunoglobulin, which is associated with severe infections. Although in subgroups of this heterogenous disease, genetic defects of molecules such as CD40-ligand or Icos have been reported, the pathogenesis of this disease in the majority of affected patients had been unresolved before this invention.

The inventors could also demonstrate for the first time that at least in a subgroup of patients this disease is associated with the presence of Bob-1-specific T-cells, which kill Bob-1-specific B-cells, which are crucial cells within the lymph nodes for the induction of a humoral immune response.

While in the above described therapeutical strategy for the treatment of tumors, lymphomas or autoimmune diseases the invention provides means and methods to induces specific T-cells, which deplete Bob-1 expressing cells, the invention also has the capacity to shut down - in other words to anergize - already existing TCs or CTLs, which have a specificity for Bob-1 protein.

This shutting down or anergizing of TCs or CTLs has the effect that the depletion of Bob-1 specific B-cells can be stopped. Accordingly, a patient recovers from an Bob-1 dependent immunodeficiency.

Accordingly, in one further embodiment the peptides, proteins, protein fragments, epitopes, sequences, composition or vaccine according to the invention can be used for treatment of Bob-1 depended immunodeficiencies by inducing anergy in naturally occurring T-cells, which are directed against the Bob-1 protein.

The term "anergy" as used herein defines one outcome, which results from a partial T-cell activation. T-cell anergy can arise either due to subtle alteration of the antigen, leading to a lower-affinity cognate interaction, or due to a lack of adequate co-stimulation. The signaling defects in anergic T-cells are partially defined, and suggest that T-cell receptor (TCR) proximal, as well as downstream defects negatively regulate the anergic T-cell's ability to be activated. Most importantly, the use of TCR-transgenic mice has provided compelling evidence that anergy is an *in vivo* phenomenon, and not merely an *in vitro* artefact. Studies in rodents and in man suggest that anergic T-cells acquire regulatory properties; the regulatory effects of anergic T cells require cell to cell contact, and appear to be mediated by inhibition of antigen-presenting cell immunogenicity. Close similarities exist between anergic T cells, and the recently defined CD4+ CD25+ population of spontaneously arising regulatory cells that serve to inhibit autoimmunity in mice.

To anergize T-cells according to the method of the invention immature or semi-mature dendritic cells presenting Bob-1-derived HLA-class I or Bob-1-derived HLA-class II-restricted epitopes are administered into patients. Immature or semi-mature dendritic cells can inactivate Bob-1-specific T-cells, as these kind of dendritic cells can anergize T-cells by the lack of costimulating molecules and by the delivery of anergizing negative signals.

Immature and semimature dendritic cells are important regulator cells protecting the body from autoimmunity. (Reviewed in Immature, semi-mature and fully mature dendritic cells: which signals induce tolerance or immunity?; M. Lutz and G. Schuler. Trends in Immunology, Volume 23, Issue 9, Page 445). In addition to direct anergisation of T-cells these dendritic cells can induce specific suppressor T-cells and regulatory T-cells. Due to their programmation by tolerogenic dendritic cells, these suppressor or regulatory T-cells secrete immunoregulatory cytokines such as IL-10 and TGF-beta, which suppress autoreactive T-cell responses. The ability of the use of semimature or immature dendritic cells to inhibit antigen-specific immune responses have been reported by several groups (for example: Lutz MB, Suri RM, Niimi M, Ogilvie AL, Kukutsch NA, Rossner S, Schuler G, Austyn JM. Immature dendritic cells generated with low doses of GM-CSF in the absence of IL-4 are maturation resistant and prolong allograft survival in vivo. Eur J Immunol. 2000 Jul; 30(7):1813-22; Madhav V. Dhodapkar and Ralph M. Steinman. Blood, 1 July 2002, Vol. 100, No. 1, pp. 174-177. Antigen-bearing immature dendritic cells induce peptide-specific CD8⁺ regulatory T-cells in vivo in humans).

Accordingly, to anergize T-cells in a further embodiment of the invention immature or semimature dendritic cells can be generated from monocytes or from hematopoetic stem cells by incubation with low doses of gm-CSF with or without incubation with IL-10. They are then incubated and thereby loaded *in vitro* with one or more of the peptides of the invention, the Bob-1 protein, fragments or epitopes thereof, a modified Bob-1 protein, fragments or epitopes thereof, or one or more nucleic acid sequence of the invention. Thereafter, according to the method to anergize T-cells in a patient the dendritic cells, have been loaded or stimulated as described above, will be applied to the patients either as intracutaneous, subcutaneous, intramuscular, intravenous or intralymphatic injection.

Such a vaccination with immature dendritic cells, which have been loaded or stimulated with peptides, proteins, protein fragments, epitopes according to the invention is used to anergize auto-reactive T-cells.

In addition, vaccination with immature dendritic cells which have been loaded or stimulated with peptides, proteins, protein fragments, epitopes according to the invention can be used to induce immunoregulatory antigen-specific suppressor T-cells. These immunoregulatory T-cells, characterized by expression of both the CD4 and the CD25 molecule, are potent suppressor of T-cell responses.

Another method to anergize T-cells is the use of antagonistic peptides, which carry modifications in comparison to Bob-1-derived peptides, leading only to partial activation of T-cells, a process which leads to anergization of Bob-1-specific T-cells. These peptides can be administered intracutaneously, subcutaneously or intramuscularly directly to patients or after loading to immature or semi-mature dendritic cells.

This method is based on a common phenomenon in T-cell activation. Modified peptides, which activate T-cell receptors below the necessary trigger threshold, can induce anergy or even apoptosis (Sloan-Lancaster, J., B.D. Evavold, P. M. Allen. 1993. Induction of T-cell anergy by altered T-cell receptor ligand on live antigen-presenting cells. *Nature 363:156.* or Brocke, S., K. Gijbels, M. Allegretta, I. Ferber, C. Piercy, T. Blankenstein, R. Martin, U. Utz, N. Karin, D. Mitchell, et al 1996. Treatment of experimental encephalomyelitis with a peptide analogue of myelin basic protein. Nature *379:343*.) Accordingly, modified Bob-1 protein, fragments or epitopes thereof are particularly useful to anergize T-cells, which are specific for Bob-1, or even to trigger apoptosis in T-cells, which are specific for Bob-1 protein expression.

### Short Description of the Figures

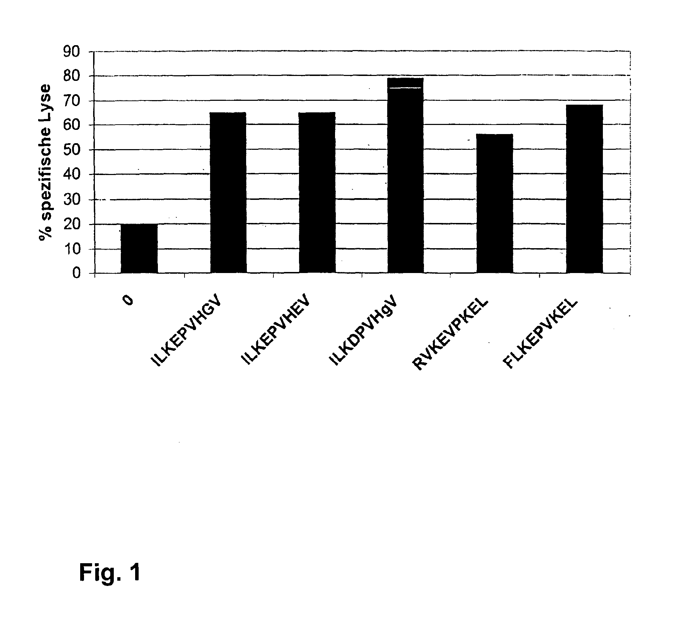

- **Figure 1**: shows the results of a chromium release assay, wherein HLA-A2-positive T2 cells were incubated with radioactive labeled chromium and different peptides. After several washing steps the CTLs, isolated from a HIV patient and stimulated for an outgrowth of CTLs with specificity to the peptide ILKDPVHGV, were incubated with the labeled and peptide presenting T2-cells and the release of chromium is measured. Specific lysis depending on the different Peptides presented is indicated. Cross-reactivity between the HIV derived peptide ILKDPVHGV and the Bob-1 derived peptide FLKEPVKEL or the modified Bob-1 derived peptide RVKEVPKEL are demonstrated.

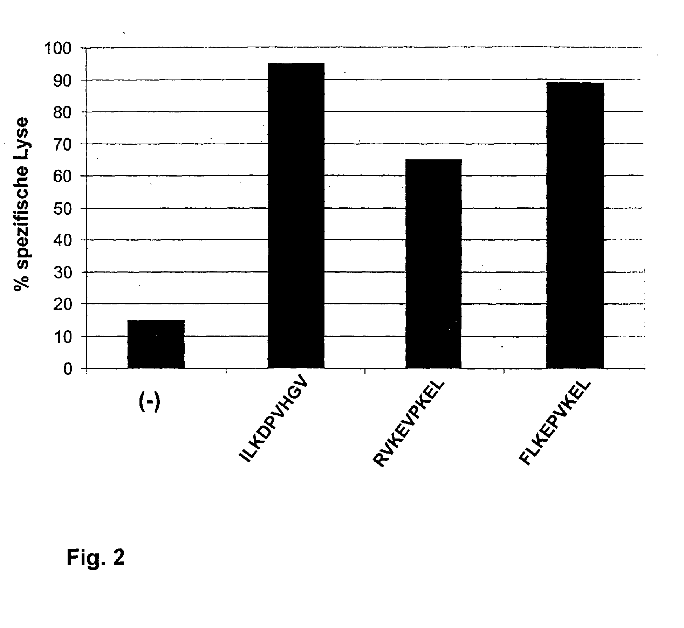

- **Figure 2**: shows the results of a chromium release assay, wherein specific HLA-A2-positive T2-cells were incubated with radioactive labeled chromium and different peptides. After several washing steps the CTLs, isolated from a patient with humoral immune defects and stimulated for an outgrowth of CTLs with specificity to the peptide ILKDPVHGV, were incubated with the labeled and peptide presenting T2-cells and the release of chromium is measured. Specific lysis depending on the different Peptides presented is indicated. Cross-reactivity between the HIV derived peptide ILKDPVHGV and the Bob-1 derived peptide FLKEPVKEL or the modified Bob-1 derived peptide RVKEVPKEL are demonstrated.

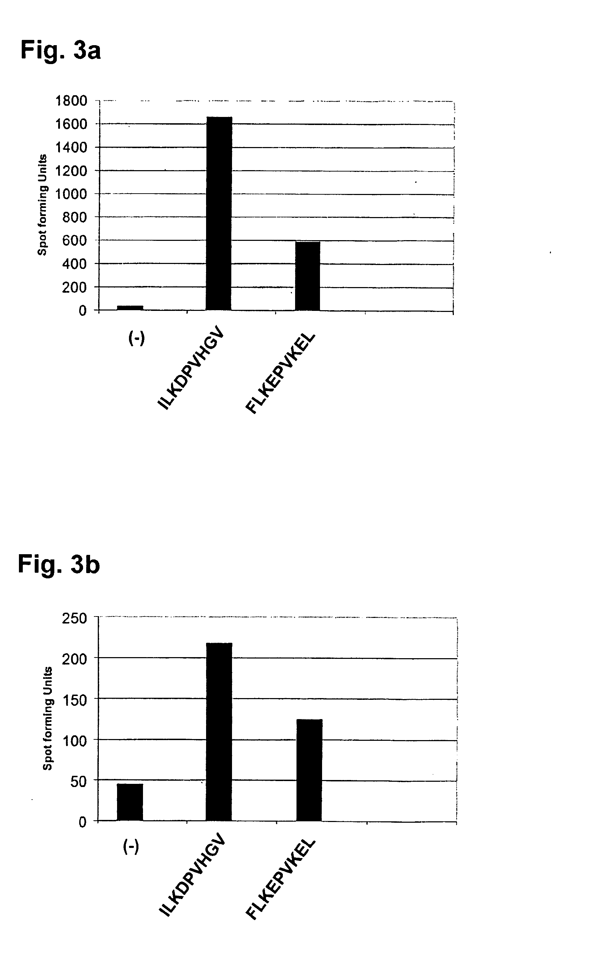

- **Figure 3a**: for an ELISPOT assay PBMC isolated from a patient with humoral immune defects were stimulated with the HIV derived peptide ILKDPVHGV and the outgrowing specific CTLs were then analyzed for their capability to specifically recognize the HIV derived peptide ILKDPVHGV and to cross-react with the Bob-1 derived peptide FLKEPVKEL.
- **Figure 3b**: for an ELISPOT assay PBMC isolated from a patient with humoral immune defects were stimulated with the Bob-1 derived peptide FLKEPVKEL and the outgrowing specific CTLs were than analyzed for their capability to specifically recognize the Bob-1 derived peptide FLKEPVKEL and to cross-react with the HIV derived peptide ILKDPVHGV.

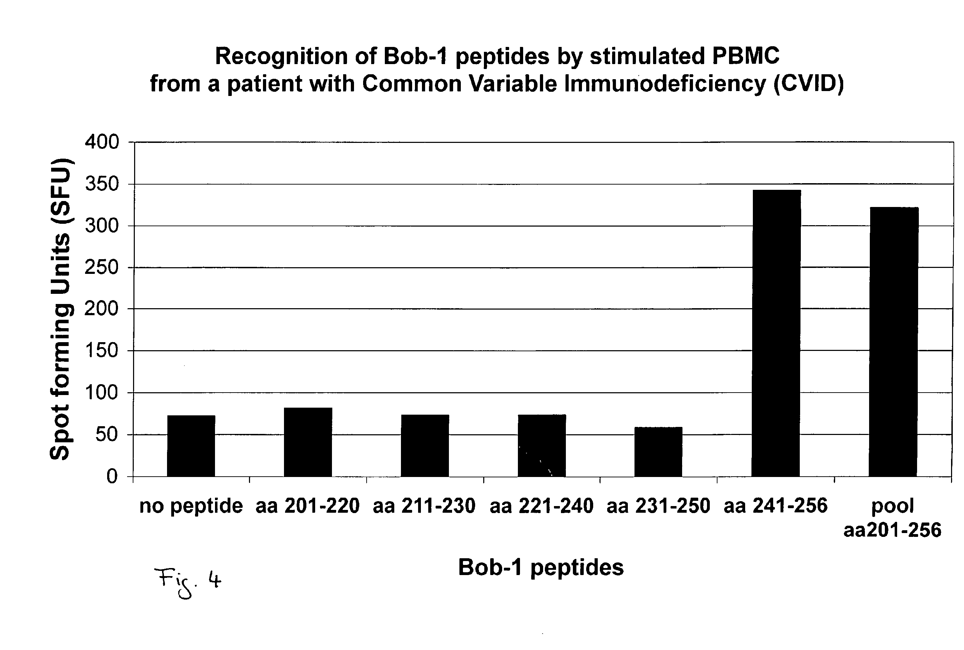

- **Figure 4**: for an ELISPOT assay PBMC isolated from an HIV-1 negative patient with common variable immunodeficiency (CVID) were stimulated with five different peptide pools containing each five 16 to 20 amino acid long peptides with a 10 amino acid overlap. These 25 peptides contained in these 5 pools were derived from a set of overlapping peptides with a 10 amino acid overlap spanning the whole human Bob-1 protein. Outgrowing specific T-cells tested in a gamma-IFN ELISPOT showed specific recognition of peptide pool 5 containing the five peptide-spanning amino acids 201 to 256 of the Bob-1 protein. Further analysis revealed that the T-cells specific for the peptide pool 5 specifically recognized the peptide DSDAYALNHTLSVEGF (amino acid 241 to 256 of Bob-1), but not the other peptides within this pool.

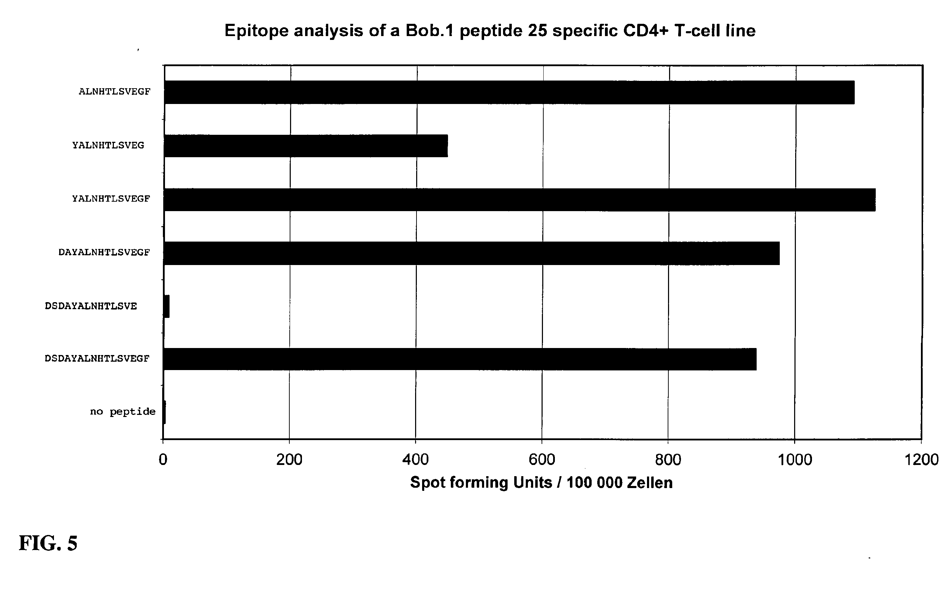

- **Figure 5**: shows an epitope analysis of Bob-specific CD4-cells from a patient with common variable immunodeficiency. PBMC from a patient with common variable immunodeficiency were stimulated with the Bob-1-peptide 25 (= DSDAYALNHTLSVEGF, corresponding to amino acids 241 to 256 of Bob.1). Outgrowing cells, which recognized specifically Bob-peptide 25 were further analyzed in a gamma-IFN-ELISPOT-assay using 50000 cells in duplicates and synthetic truncated peptides. This experiment demonstrates that the Bob-specific cell line specifically recognizes the peptide ALNHTLSVEGF, but less efficient peptides with omission of the C-terminal F, and no peptides with omission of the C-terminal G.

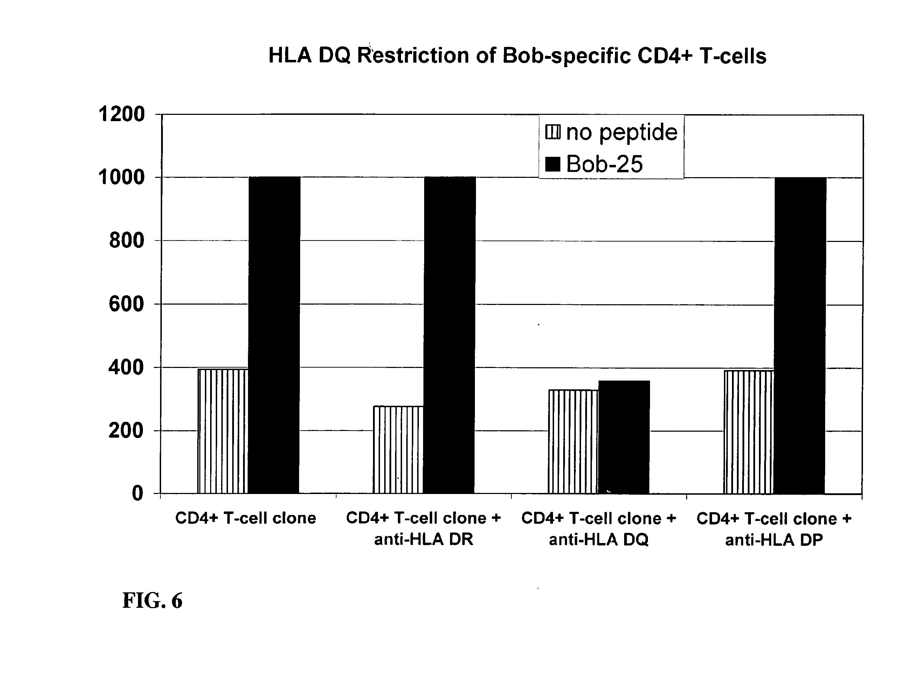

- **Figure 6**: shows an epitope analysis of Bob-specific CD4-cells from a patient with common variable immunodeficiency. PBMC from a patient with common variable immunodeficiency were stimulated with the Bob-peptide 25 (= DSDAYALNHTLSVEGF, corresponding to amino acids 241 to 256 of Bob). Outgrowing cells recognizing specifically Bob-peptide 25 were further analyzed in a gamma-IFN-ELISPOT-assay using 50000 cells in duplicates and autologous EBV-transformed B-cells (B-LCL) incubated with and without Bob-peptide 25 and antibodies against HLA class II DR, DQ or DP. Bob-25 induced gamma-Interferon production was inhibited by incubation with antibodies against HLA DQ, but not with antibodies against HLA DR and DP molecules. This demonstrates that Bob-peptide 25 is presented by HLA DQ.

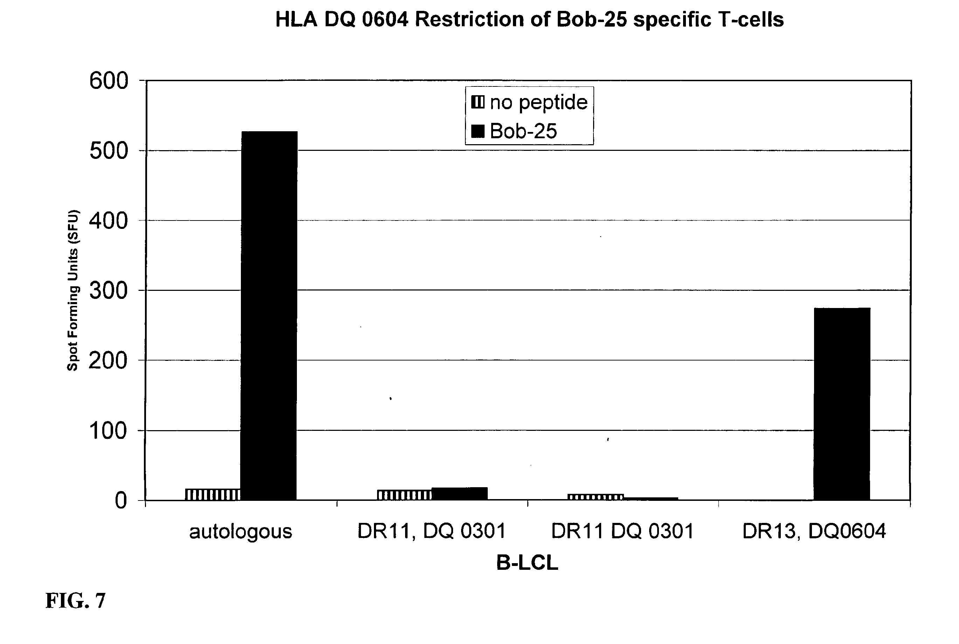

- **Figure 7**: shows an epitope analysis of Bob-specific CD4-cells from a patient with common variable immunodeficiency. PBMC from a patient with common variable immunodeficiency were stimulated with the Bob-peptide 25 (= DSDAYALNHTLSVEGF, corresponding to amino acids 241 to 256 of Bob). The HLA class II type of that patient is: DR11, DR13, DQ0301, DQ0604. Outgrowing cells recognizing specifically Bob-peptide 25 were further analyzed in a gamma-IFN-ELISPOT-assay using 50000 cells in duplicates and autologous and HLA-class II-matched EBV-transformed B-cells (B-LCL) incubated with and without Bob-peptide 25. Bob-25 induced gamma-Interferon production was stimulated only by Bob-peptide 25 - incubated B-cell lines expressing DQ0604, but not by B-cell lines expressing DQ 0301 or DR11. This demonstrates that the HLA DQ0604 molecule can specifically bind and present the Bob-peptide 25 to specific CD4+ T-cells in patients with common variable immunodeficiency.

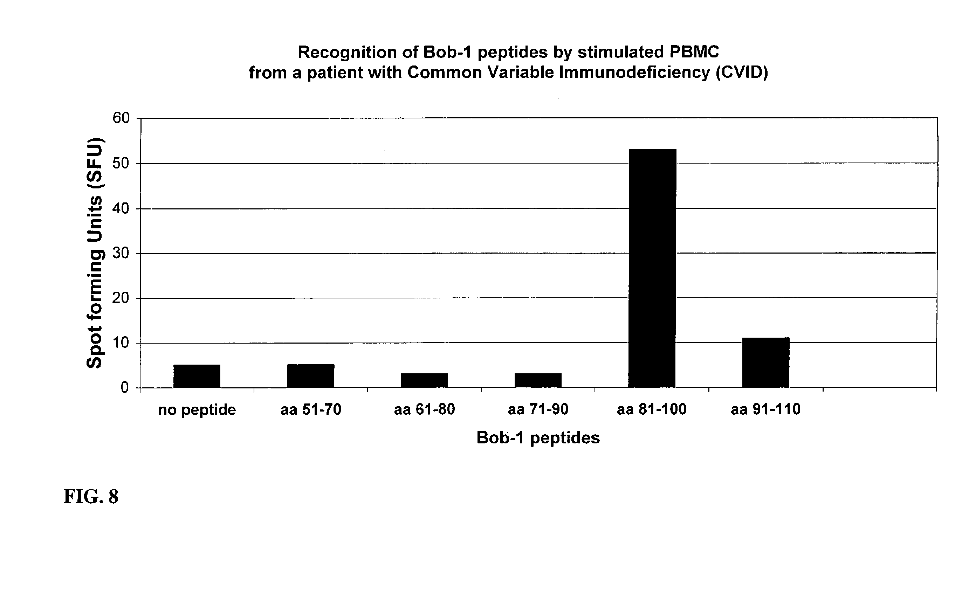

- **Figure 8**: shows an epitope analysis of Bob-specific CD4-cells from a patient with common variable immunodeficiency. PBMC from a patient with common variable immunodeficiency were stimulated with a peptide pool containing five 20 amino acid (aa) long peptides with a 10 amino acid long overlap, corresponding to amino acids 51 to 110 of Bob-1. Outgrowing cells recognized specifically Bob-peptide aa 81-100 in a gamma-IFN-ELISPOT-assay using 50000 cells in duplicates and synthetic truncated peptides. This experiment demonstrates that the Bob-specific cell line specifically recognizes the peptide AALCAGWLSQPTPATLQPLA (corresponding to amino acids 81 - 100).

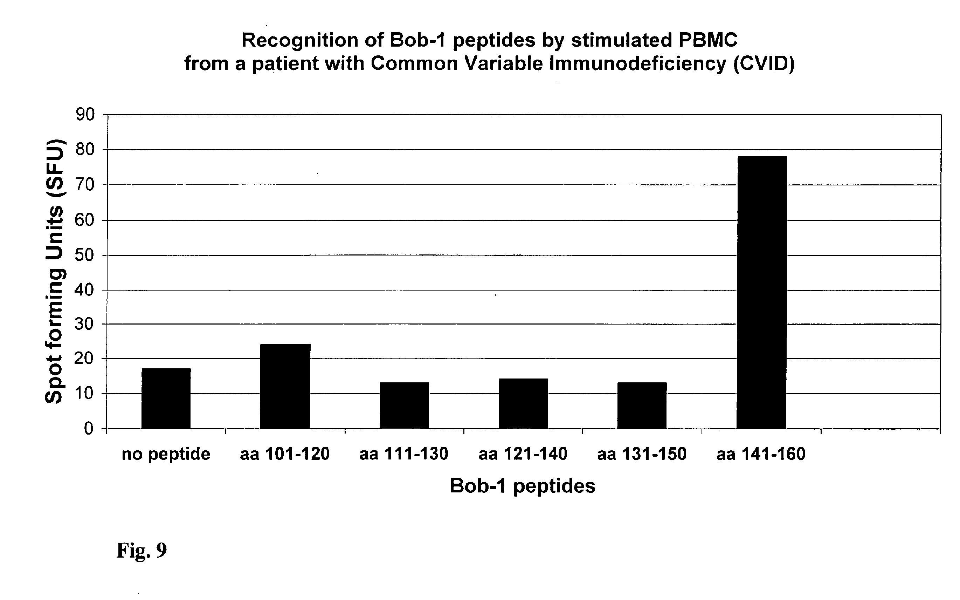

- **Figure 9**: shows an epitope analysis of Bob-specific CD4-cells from a patient with common variable immunodeficiency. PBMC from a patient with common variable immunodeficiency were stimulated with a peptide pool containing five 20 amino acid (aa) long peptides with a 10 amino acid long overlap, corresponding to amino acids 101 to 160 of Bob-1. Outgrowing cells recognized specifically Bob-peptide 141-160 in a gamma-IFN-ELISPOT-assay using 50000 cells in duplicates and synthetic truncated peptides. This experiment demonstrates that the Bob-specific cell line specifically recognizes the peptide TYASPPLITNVTTRSSATPA (corresponding to amino acids 141 - 160).

### Examples

### I. Identification and isolation of Bob-1 cross-reactive T-cells from HIV-1 infected patients.

Using an T-cell analyzing assay the inventors were able to identify in several HIV-1 infected patients T-cells, which recognized specifically the HIV-1 specific T-cell epitope ILKEPHGV or naturally occurring variants therefore, but at the same time showed a cross-reactivity and significant specificity for one or more Bob-1 epitopes.

In addition, the inventors were able to identify also in other patients without HIV-1 infections and HLA negative, which were suffering from the common variable immunodeficiency, Bob-1 - specific T-cells which recognized the epitope RVKEVPKEL and other epitopes within Bob-1. Epitope RVKEVPKEL was also recognized by HLA A2 negative patients. This indicates that this epitope can be recognized also by other epitopes than HLA A2.

The preferred T-cell analyzing assay was the ELISPOT assay, which has evolved to a standard assay for the detection of antigen-specific T-cells in immunology. ELISPOT assays were conducted using R5AB media consisting of RPMI 1640 medium with supplements and 5 % human AB serum (Sigma-Aldrich, Steinheim, Germany). Both freshly isolated lymphocytes from peripheral blood (PBMC) or from lymph nodes and peptide stimulated PBMC were used. Nitrocellulose filter-backed microtiter plates (96 well; MAHA-S-4510, Millipore, Molsheim, Germany) were coated with 50 µl of interferon-γ antibody 1-D1K (Mabtech, Stockholm, Sweden) at a concentration of 10 µg/ml. After four washes with PBS and blocking with R5AB, 5 x 10⁵ PBMC were added in 100 µl R5AB to the precoated wells in duplicate. Peptides were added directly to the wells at a final concentration of 40 µg/ml and the plates were incubated for 10 h at 37°C in 5% CO₂. After six washes with PBS containing 0.05% Tween 20 (PBS/T 0.05%), 100µl biotinylated interferon-γ monoclonal antibody 7-B6-1 (Mabtech) was incubated at a final concentration of 2µg/ml for 2h at 37°C in 5% CO₂. After washing with 0.05% PBS/T, 100µl avidin/peroxidase solution (Vectastain ABC-Kit, Vector Laboratories, CA, USA) was added to each well. After incubation for 1 h at room temperature and after three washes with 0.05% PBS/T and three washes with PBS, 100 µl AEC substrate (3-amino-9-ethylcar-bazole, Sigma-Aldrich) containing 0.01% H₂O was added as chromogen and spots developed within 4 min. The colorimetric reaction was stopped by washing the plates with distilled water and the plates were then air dried. Spots were counted using a video-based automatic ELISPOT reader (AID Autoimmun Diagnostika, Strassberg, Germany).

In a chromium release assay it was additionally shown that these Bob-1 cross-reactive CTL were able to specifically bind and destroy Bob-1-expressing, HIV-negative cells.

For this, PBMC were sensitized with synthetic peptides of the invention at a final concentration of 2 µg/ml in 1ml R 10 medium supplemented with 10 U/ml interleukin-2. After 2 weeks, outgrowing cells were tested for specific recognition of peptide-pulsed PBMC in a standard chromium-release assay. Peptide-specific CTL lines were restimulated every 2 weeks with peptide-pulsed irradiated PBMC (irradiation with 60 Gy) and irradiated allogenic feeder cells (irradiation with 40 Gy).

### II. Identification and isolation of Bob-1 cross-reactive T-cells from a patient having a lack of immunoglobulin.

Using a T-cell analyzing assay the inventors were able to identify in patients having a lack of immunoglobulin (Ig) T-cells, which showed a significant specificity for one or more Bob-1 epitopes.

In a chromium release assay it was subsequently shown that these Bob-1 specific T-cells were able to specifically bind and destroy autologous B-cells, which had been transformed by Epstein Barr virus (EBV). This method allowed for the first time a diagnostic tool to identify and characterize patients, who suffer from an auto-immunogenic, T-cell-induced depletion of Bob-1 expressing B-cells. This newly discovered autoimmune disorder was named "Bob-1 associated T-cell autoimmunity".

In the same context it was shown that this autoimmune reaction against Bob-1 expressing B-cells was correlated with the reduction of the Ig level in the patients. Accordingly, it was shown that an autoimmune reaction against Bob-1 protein could cause a humoral immune defect, characterized *iter alia* by the destruction of follicular B-cells. While, however, the number of peripheral B-cells can be quite normal in such patients, they have a reduced level of immunoglobulin. This can be explained by the higher expression of Bob-1 in follicular center B-cells in comparison to resting circulating B-cells. The progressive decrease of immunoglobulins is associated in many patients with the emergence of infectious complications. By the discovery of the Bob-1 associated T-cell autoimmunity the pathogenesis of at least a subgroup of patients with this diseases, commonly named as "Common variable Immunodeficiency" has been for the first time explained by the inventors.

The invention not only contributes to the exploration of the pathogenesis of patients with the "Common Variable Immunodeficiency Syndrome", but also to other important diseases such as "MGUS" (Monoclonal Gammopathy of Unclear Significance) and Plasmocytoma (Multiple Myeloma).

Patients with MGUS and Plasmocytoma have a paraprotein in their serum which is a monoclonal immunoglobulin derived from an expanded B-cell clone. Patients with MGUS, which is a common disorder predominantly in elderly persons with a prevalence of up to 3%, have a significant risk to develop a plasmocytoma which is a malignant tumor classified as a Non-Hodgkin-Lymphoma.

The inventors could demonstrate that MGUS and plasmocytoma can emerge in patients with Bob-1 associated T-cell autoimmunity. The destruction of normal Bob-1-expressing follicular center B-cells by autoreactive T-cells leads to dysregulation of the B-cell immune response and depletion of the B-cell zones within the lymphatic organs thus providing the opportunity for the development and clonal expansion of genetically damaged and modified B-cell clones which are resistant against the Bob-1 specific CTL activity. The invention provides important insight into the pathogenesis of MGUS and plasmocatoma as the commonly observed lack of normal immunoglobulins in these disorders is not the consequence of the expansion of a monoclonal B-cell population, but in contrast, the reduction of follicular B-cells by autoreactive B-cell is the pre-requisite of the development of MGUS and plasmocytoma.

As a consequence of these facts, it is considered a potential strategy to treat CVID, MGUS and plasmocytoma by anergizing or inhibiting Bob-1 specific TCs or CTLs according to the methods of the invention. Under these circumstances normal B-cell populations could recover which would restore production of normal immunoglobulins and which would prevent further expansion of monoclonal B-cell clones.

### III. Chromium release assay to test the specificity of TC induction with different Peptides.

T-cells isolated from a HIV patient were peptide stimulated with the peptide ILKDPVHGV and small doses of interleukin 2. Outgrowing CTLs were then tested in a chromium release assay for their peptide specific lyses.

For this specific HLA-A2-positive T2-cells, which are a fusion between a B-cell and a T-cell having a TAP-transporter defect and therefore not being capable to present internal Bob-1 protein, were incubated with radioactive labeled chromium and different peptides. Subsequently, after several washing steps the CTLs were incubated with the labeled and peptide presenting T2-cells and the release of chromium is measured.

As is shown in Figure 1, the CTLs isolated from the HIV patient specifically recognizes and destroys peptide presenting T2 cells.

It could be shown that the peptides deriving from a modified Bob-1 protein showed better results compared with peptides derived from normal Bob-1 protein. This is due to the fact that the modified peptides were optimized by the inventors for binding to the HLA A2-molecule.

### IV. Chromium release assay to test the specificity of TC induction with different peptides.

T-cells isolated from a patient with a humoral immune defect were peptide stimulated with the HIV derived peptide ILKDPVHGV and small doses of interleukin 2. Outgrowing CTLs were then tested in a 5-hour chromium release assay for their peptide specific lyses.

For this specific HLA-A2-positive T2-cells, which are a fusion between a B-cell and a T-cell having a TAP-transporter defect and therefore not being capable to present internal Bob-1 protein, were incubated with radioactive labeled chromium and different peptides. Subsequently, after several washing steps the outgrown CTLs were incubated with the labeled and peptide presenting T2-cells and the release of chromium is measured.

As is shown in Figure 2, the CTLs isolated from the patient specifically recognize T2 cells presenting the HIV derived peptide ILKDPVHGV and furthermore show high cross-reactivity to the Bob-1 derived peptide RVKEVPKEL or the modified Bob-1 derived peptide FLKEPVKEL.

### V. ELISPOT assay to analyze cross-reactivity of induced CTL by different peptides

For the one ELISPOT assay (compare to Figure 3a) PBMC isolated from a patient with humoral immune defects were stimulated with the HIV derived peptide ILKDPVHGV and the outgrowing specific CTLs were than analyzed for their capability to specifically recognize the HIV derived peptide ILKDPVHGV and furthermore to cross-react with the Bob-1 derived peptide FLKEPVKEL. For the second ELISPOT assay (compare to Figure 3b) PBMC isolated from a patient with humoral immune defects were stimulated with the Bob-1 derived peptide FLKEPVKEL and the outgrowing specific CTLs were than analyzed for their capability to specifically recognize the Bob-1 derived peptide FLKEPVKEL and to cross-react with the HIV derived peptide ILKDPVHGV.

The results clearly indicate that there is a strong cross-reactivity between the Bob-1 derived peptide FLKEPVKEL and the HIV derived peptide ILKDPVHGV.

### VI: Isolation and characterization of Bob-1 specific CD4 T-helper cells from patients with common variable immunodeficiency.

In HIV-1 negative patients with common variable immunodeficiency the inventors were able to identify Bob-1 specific T-cells. For example, CD4⁺ T-helper cells derived from patients with this immunodeficiency specifically recognized the Bob-1 derived peptide DSDAYALNHTLSVEGF.

PBMC from patients with common variable immunodeficiency, characterized by repeated infections due to reduced serum levels of IgG, IgA and IgM, were stimulated with pools of overlapping peptides spanning the whole Bob-1 protein. Each of the five peptide pools contained five 16 to 20 amino acid long peptides overlapping by 10 amino acids. Outgrowing cells analyzed by a gamma-IFN ELISPOT showed a specific recognition of peptide pool 5 containing five peptides corresponding to amino acids 201 to 256 of the Bob-1 protein (Figure 4).

Further analysis with individual peptides demonstrated recognition of peptide 25 with the sequence DSDAYALNHTLSVEGF corresponding to amino acids 241 to 256 of the Bob-1 protein.

In further experiments CD4+ or CD8+ T-cells were depleted from DSDAYALNHTLSVEGF specific cell lines by CD4 or CD8 specific monoclonal antibodies linked to magnetic beads using a standard magnetic cell separation technique (Eays Sep™, Stem Cell Technologies, Vancouver,BC).

This experiments clearly demonstrated that the Bob-1 peptide-25-specific T-cells from this patient were CD4 T-helper cells.

In further ELISPOT - experiments DSDAYALNHTLSVEGF specific cell lines were further characterized by truncated peptides. In these experiments 50000 cells were incubated with truncated peptides overnight and analyzed for gamma-IFN - production in a standard ELISPOT assay. These experiments demonstrated that DSDAYALNHTLSVEGF specific cells still could recognize the peptide ALNHTLSVEGF, whereas the peptide YALNHTLSVEG is recognized less well and peptides with omission of the C-terminal G are not recognized, indicating that the optimal epitope is the peptide ALNHTLSVEGF (aa 246- aa 256) (Figure 5). Further experiments characterized the HLA type of the DSDAYALNHTLSVEGF specific cells (Figure 6 and 7).

In the same patient stimulation of PBMC with peptide pool 2 containing five 20 amino acid long peptides spanning amino acid 51 to 110) revealed by ELISPOT analysis the outgrow of T-cells specifically recognizing peptide aa 81 - aa 100 (Figure 8).

Stimulation of PBMC with peptide pool 3 containing five 20 amino acid long peptides spanning amino acid 101 to 160) revealed by ELISPOT-analysis the outgrow of T-cells specifically recognizing peptide aa141-160 (Figure 9).

What is most astonishing is that some of the patients were cured from their rheumatic auto-immune disease, most probably by the induction of Bob-1 specific T-cells, particularly DSDAYALNHTLSVEGF specific cells.

In addition, such patients also recognized other Bob-1 derived peptides such as TYASPPLITNVTTRSSATPA and AALCAGWLSQPTPATLQPLA, which indicates that also these peptides are useful for the treatment of Bob-1 dependent diseases or disorders, such as tumors, lymphomas or auto-immune diseases.

## Claims

1. Peptides for the identification, isolation and/or induction of Bob-1 specific and/or Bob-1 cross-reactive T-cells.

2. Peptides for the identification, isolation and/or induction of Bob-1 specific and/or Bob-1 cross-reactive T-cells, whereby the Peptide has the general amino acid sequence
X₁ X₂ K X₃ P X₄ X₅ X₆ X₇
whereby
- X₁ is selected from the group comprising any naturally occurring amino acid, particularly Arginine (R), Isoleucine (I), Phenylalanine (F), Tyrosine (T) or Serine (S) is,
- X₂ is Methionine (M), Valine (V), Leucine (L) or Isoleucine (I),
- X₃ is Glutamic acid (E) or Aspartic acid (D),
- X₄ is selected from the group comprising any naturally occurring amino acid particularly Valine (V), Leucine (L), Isoleucine (I), or Alanine (A) is,
- X₅ is selected from the group comprising any naturally occurring amino acid particularly Histidine (H) or Leucine (L),
- X₆ is selected from the group comprising any naturally occurring amino acid particularly Glutamic acid (E) or Glycine (G), and
- X₇ is Methionine (M), Valine (V), Leucine (L), Isoleucine (I) or Alanine (A).

3. Peptid according to claim 1 or 2 selected from the group comprising the peptides FLKEPVKEV, SLKEPVKEL, YLKEPVKEL, FLKEPVKEL, ILKEPVHEV, ILKEPVHGV, RVKEPVKEL, DSDAYALNHTLSVEGF, AYALNHTLSVEGF, YALNHTLSVEGF, YALNHTLSVEG, ALNHTLSVEGF, TYASPPLITNVTTRSSATPA or AALCAGWLSQPTPATLQPLA.

4. Peptide according to any of the claims 1 to 3, wherein the peptide is a fragment of nine or more amino acids and/or a modified version of the amino acid sequence DSDAYALNHTLSVEGF, TYASPPLITNVTTRSSATPA or AALCAGWLSQPTPATLQPLA.

5. Nucleic acid sequence encoding alone or in combination one or more of the peptides according to any of the claims 1 to 4.

6. Isolated T-cells specific and/or cross-reactive with a peptide according to any of the claims 1 to 4, a Bob-1 protein, a fragment and/or epitopes thereof.

7. T-cells according to claim 6, whereby the T-cells are cytotoxic.

8. T-cells according to claim 6, whereby the T-cells are CD4⁺ T-helper cells.

9. Peptides according to the any of the claims 1 to 4, the nucleic acid sequence according to claim 5 or T-cells according to the claims 6, 7 or 8 as a medicament or a vaccine.

10. Peptides according to the any of the claims 1 to 4, the nucleic acid sequence according to claim 5, or T-cells according to the claims 6, 7 or 8, for the use in the treatment of malignant lymphomas, Bobo-1 expressing tumors, auto-immune diseases and/or any disease or disorder correlated with the expression or overexpression of Bob-1 protein.

11. Composition comprising the peptides according to any of the claims 1 to 4, the nucleic acid sequence according to claim 5, or T-cells according to the claims 6, 7 or 8 and a pharmaceutical acceptable carrier and/or diluent.

12. Vaccine comprising the peptides according to any of the claims 1 to 4, the nucleic acid sequence according to claim 5, or T-cells according to the claims 6, 7 or 8 for the induction of Bob-1 specific and/or Bob-1 cross-reactive T-cells.

13. Method to increase *in vitro* in a T-cell population the number of T-cells, which are specific and/or cross reactive for Bob-1 protein, fragments or epitopes thereof, or at least one peptide according to any of the claims 1 to 4, comprising
- contacting a population of PBMC with a Bob-1 protein, fragment or epitope thereof, or at least one peptide according to claims 1, 2, 3 or 4;
- optionally, adding immune hormones to the reaction;
- analyzing with appropriated assays the induction of T-cell growth in the stimulated population, whereby the preferred assay is an ELISPOT, a chromium-release assay or direct visualization of peptide specific T-cells by soluble HLA-peptide complexes such as HLA tetramers; and, optionally,
- isolating stimulated T-cells, which are specific and/or cross reactive for Bob-1 protein, fragments or epitopes thereof.

14. Method to diagnose diseases or disorders correlated with a Bob-1 expression comprising
- isolating PBMC from a patient
- contacting the PBMC with a Bob-1 protein, a fragment or epitope thereof, or at least one peptide according to any of the claims 1 to 4;
- analyzing in an appropriated assays the amount of the Bob-1 specific and/or Bob-1 cross-reactive T-cell, whereby the preferred assays are an ELISPOT assay, a chromium-release assay, an intracellular or extracellular cytokine staining of T-cells after antigen-stimulation or direct visualization of specific T-cells by soluble HLA-peptide complexes such as HLA - tetramers; and
- analyzing and comparing further physiological parameters and the amount of Bob-1 specific or cross-reactive T-cells.

15. Diagnostic kit comprising a container and at least one of the peptides according to any of the claims 1 to 4, at least one of the nucleic acid sequence according to claim 5, orat least one of the T-cells according to the claims 6, 7 or 8.

16. Use of Bob-1 protein, fragments or epitopes thereof, modified Bob-1 protein, fragments or epitopes thereof, any of the peptides according to claims 1, 2, 3 or 4 and/or the nucleic acid sequence according to the claims 5 for the induction of T-cells according to the claims 6, 7 or 8 or the method according to claim 13 or 14.

17. Use of Bob-1 protein, fragments or epitopes thereof, modified Bob-1 protein, fragments or epitopes thereof, and/or any of the peptides according to claims 1, 2, 3 or 4 for the diagnosis of diseases or disorders correlated with the expression of Bob-1 protein.

18. Method of treating Bob-1 expressing lymphomas or other Bob-1 expressing tumors in patients in need thereof comprising
- administering to the patient activated Bob-1 specific and/or cross-reactive T-cells, whereby the T-cells have been induced and activated *ex vivo* by the incubation with Bob-1 protein, fragments or epitopes thereof, modified Bob-1 protein, fragments or epitopes thereof and/or any of the peptides according to claims 1, 2, 3 or 4; and, optionally,
- administering immunoglobulin as an additional replacement therapy.

19. Method of treating Bob-1 expressing lymphomas or other Bob-1 expressing tumors in patients in need thereof comprising
- administering a vaccine according to claim 12 for induction of Bob-1 specific T-cells; and, optionally,
- administering immunoglobulin as an additional replacement therapy.

20. Method of treating auto-immune diseases, auto-immunopathies and/or allergies in patients in need thereof comprising
- administering to the patient activated Bob-1 specific and/or cross-reactive T-cells, whereby the activated T-cells have been induced and activated *ex* *vivo* by the incubation with Bob-1 protein, fragments or epitopes thereof, modified Bob-1 protein, fragments or epitopes thereof and/or any of the peptides according to claims 1, 2, 3 or 4; and, optionally,
- administering immunoglobulin as an additional replacement therapy.

21. Method of treating auto-immune diseases, auto-immunopathies and/or allergies in patients in need thereof comprising
- administering a vaccine according to claim 12 for induction of Bob-1 specific T-cells; and, optionally,
- administering immunoglobulin as an additional replacement therapy.

22. Method to induce an immune reaction in a living animal or human, comprising
- administering to the living animal or human a Bob-1 protein, a fragment or an epitope thereof, modified Bob-1 protein, fragments or epitopes thereof, at least one peptide according to claims 1, 2, 3 or 4, at least one nucleic acid sequence according to the claim 5 and/or the vaccine according to claim 12; and, optionally,
- administering immune hormones, adjuvants or factors for enhancement of the immune response to the living animal or human.

23. Method of treating Bob-1 associated auto-immune diseases particularly common variable immunodeficiency, comprising
- administering to the living animal or human immature or semimature dendritic cells incubated or transduced with Bob-1 protein, fragments or epitopes thereof, modified Bob-1 protein, fragments or epitops thereof, at least one peptide according to claims 1, 2, 3 or 4, at least one nucleic acid sequence according to the claim 5 and/or the vaccine according to claim 12.

24. Method of treating Bob-1 associated auto-immune diseases particularly common variable immunodeficiency, comprising
- administering to the living animal or human immature or semimature dendritic cells incubated or transduced with modified peptides, which acted as peptide ligands derived from a modified Bob-1 protein, a fragment or an epitope thereof, or at least one modified peptide from the peptide according to claims 1, 2, 3 or 4, at least one modified nucleic acid sequence according to the claim 5.
